# EUROPEAN PATENT SPECIFICATION

(11) **EP 3 946 247 B1**
(45) Date of publication and mention of the grant of the patent: **11.09.2024**
(21) Application number: 20782965.6
(22) Date of filing: 01.04.2020
(51) Int. Cl.: A61K 8/81, A61K 8/31, A61K 8/06, A61Q 19/00, A61Q 19/10, A61Q 5/02, A61K 8/04, A61K 8/49, A61K 8/69, A61K 8/86

(54) **PERSONAL CARE COMPOSITIONS AND METHODS COMPRISING TRANS-1-CHLORO-3, 3, 3-TRIFLUOROPROPENE AND TRANS-1, 3, 3, 3-TETRAFLUOROPROPENE**
KÖRPERPFLEGEMITTEL UND VERFAHREN ENTHALTEND TRANS-1-CHLOR-3, 3, 3-TRIFLUORPROPEN UND TRANS-1, 3, 3, 3-TETRAFLUORPROPEN
COMPOSITIONS ET PROCÉDÉS DE SOINS PERSONNELS COMPRENANT DU TRANS-1-CHLORO-3, 3, 3-TRIFLUOROPROPÈNE ET DU TRANS-1, 3, 3, 3-TETRAFLUOROPROPÈNE

(30) Priority: 01.04.2019 US 201962827714 P; 20.03.2020 WO PCT/CN2020/080355
(43) Date of publication of application: 09.02.2022
(73) Proprietor: Honeywell International Inc., Morris Plains, NJ 07950 (US)
(72) Inventor: LING, Yiu Keung, Charlotte, North Carolina 28202 (US); SETIAWAN, Barry, Charlotte, North Carolina 28202 (US); WANG, Changming, Charlotte, North Carolina 28202 (US)
(74) Representative: Stepney, Gregory John
(86) International application number: PCT/CN2020/082758
(87) International publication number: WO 2020/200239

(56) References cited:
- WO-A1-2012/158870
- WO-A1-2018/075861
- WO-A1-2018/075861
- WO-A2-2012/068572
- CN-A- 109 464 300
- US-A1- 2017 313 841
- US-A1- 2018 179 128
- US-A1- 2019 020 301

## Description

### CROSS REFERENCE TO RELATED APPLICATIONS

The present application relates to and claims the priority benefit of US provisional application 62/827,714, filed April 2, 2019, which is incorporated herein by reference in its entirety.

The present application also relates to and claims the priority benefit of PCT Application No. PCT/CN2020/080355, filed March 20, 2020, which is incorporated herein by reference in its entirety.

### FIELD OF THE INVENTION

The present invention relates to personal care compositions and to methods and devices for providing personal care, including but not limited to compositions, articles and methods that provide novel foam forms and/or which provide novel foaming effects.

### BACKGROUND

Personal care compositions, such as skin lotions and cleansers and shaving creams (including foams and gels), generally comprise several components in addition to the active ingredient. Such additional ingredients may include, for example, thickeners, suspending agents, surfactants and foaming agents, which are included in order to effect a property of the composition as it is being dispensed and/or upon application to the skin.

One particular type of formulation is a gel which is self-foaming either upon being dispensed and/or upon being rubbed into or onto the area of the skin or scalp being treated. For example, one of the more popular forms of personal care products used today is the post-foaming shave gel. In such post-foaming compositions, it is common to add soaps, surfactants, active ingredients and other components with relatively volatile hydrocarbons in an effort to form clear, stable gels or emulsions when kept under pressure. Once these materials are dispensed and mechanically agitated, the gels form foams, generally due to the post-dispensing addition of energy to the gel.

Applicants have come to appreciate that the performance of prior personal care formulations, including post-foaming and non post-foaming formulations, is lacking and/or could be improved in several respects. By way of example, applicants have found that in many prior formulations the volatile hydrocarbons that were heretofore used to form the foam in post-foam applications resulted in the degradation of other desirable aspects of the formulation. For example, applicants have come to appreciate that such prior post-foam formulations produced a foam too quickly after application, which tends to negate the ability of the formulation to spread on the skin or hair of the user, and/or to penetrate into the skin/hair to the extent that would be desirable to the user. Furthermore, even to the relatively small extent that prior post-foam formulations were able to spread on the skin prior to foaming, such formulations did not enhance or improve the ability of the active ingredient to penetrate into the pores of the skin and thereby provide both the perception and the actuality of improved performance.

Furthermore, applicants have come appreciate that in many prior formulations the look and/or initial sensation on the skin/scalp is not as attractive to the user as could be achieved and detracts substantially from the satisfaction of the user when the product is used for its intended purpose. For example, applicants have come appreciate that if a foam or gel that is otherwise acceptable for its intended purpose but appears when dispensed to drippy, soggy and/or otherwise unattractive, the success of the product could be unacceptable or at least less than desired.

Another aspect of prior formulations has been the ability to provide the user with the sensation of cooling as a result of application of the personal care product to the skin and/or hair. Such sensation has been provided in many prior formulations by the inclusion of certain ingredients known as sensates, which manipulate the transient receptor potential (TRP) channels contained in human tissue. See, for example, US 2017/0000713. Applicants have come to appreciate that the use of such sensate ingredients can have several disadvantages, including relatively high cost of these ingredients and their derivatives. Another potential disadvantage is the need for sensates to interact directly with the biological function of the user, which can be undesirable in certain cases/applications, such as users who may be especially sensitive to and/or allergic to the direct biological interaction and who might experience longer-lasting side effects as a result of the ingredient remaining in contact with the user. Accordingly, applicants have come to appreciate the desirability of achieving such a cooling sensation using a simpler and less costly mechanism and without the above-noted other disadvantages of sensates. Furthermore, applicants have come to appreciate the desire of many users to experience the cooling sensation that appears essentially immediately upon application to the skin/hair/mucous membrane, and these sensates have limited use in those skin, body and mucous membrane applications where an apparently instant refreshing cooling sensation is desired. Moreover, applicants have also come to appreciate the advantage of treatment compositions and methods in which the cooling effect is long lasting and preferably at the same time also being perceived to provide essentially immediate cooling sensation upon application.

Applicants have also come to appreciate that the use of certain materials, as described hereinafter, to form the foam can have advantages in the production of a desirable and/or novel foam forms. However, applicants have also come to appreciate that in many such cases the ability to maintain the components in a stable form while in the container under pressure can be a substantial challenge with non-predictable results.

### BRIEF DESCRIPTION OF THE DRAWING

The patent or application file contains at least one drawing executed in color. Copies of this patent or patent application publication with color drawing(s) will be provided by the Office upon request and payment of the necessary fee.
Figure 1 is a color photograph showing the foams produced according to the experiment described in Example 1A.
Figure 2 is a color photograph showing the foams produced according to the experiment described in Example 2A.
Figure 3 is a color photograph showing the foams produced according to the experiment described in Examples 3A through 3D.

### SUMMARY

Applicants have come to appreciate that one or more of the disadvantages and failures described above in connection with prior formulations and methods, and preferably all of such disadvantages and failures, can be reduced or substantially eliminated using the methods, articles and personal care formulations according to the present invention. In addition and/or in the alternative, applicants have developed highly desirable and/or novel formulations that were not heretofore known or available.

The present invention includes methods of treating human skin, hair and/or mucous membranes comprising:
(a) providing a personal care composition in the form of a gel, lotion or cream or the like comprising: (i) one or more active ingredients; (ii) optionally but preferably one or more auxiliary gel, lotion or cream forming components; and (iii) foam forming component(s) comprising trans-1-chloro-3,3,3-trifluoropropene (1233zd(E)) and trans-1-3,3,3-tetrafluoropropene (1234ze(E));
(b) spreading said gel, lotion or cream on and/or into the skin, hair and/or mucous membrane of the human being treated; and
(c) foaming said gel, lotion or cream during delivery and/or while in contact with the skin, hair and/or mucous membrane by evaporating said 1233zd(E)) and said 1234ze(E) from said gel, lotion or cream. For the purposes of convenience, methods according to the present invention is referred to herein as **Method 1.**

The present invention also includes methods of treating human skin, hair and/or mucous membranes comprising:
(a) providing a personal care composition in a container under pressure in the form of a stable oil-in-water emulsion comprising:
   (i) a continuous aqueous phase comprising water;
   (ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise from about 40% to about 60% by weight of said personal care composition and are present in a trans1233zd:trans1234ze weight ratio of from about 8:1 to about 1:1;
   (iii) an emulsifier having an HLB value of from about 7 to about 18 and which may be present in the continuous and/or discontinuous phase;
   (iv) one or more active ingredients which may be present in the continuous and/or discontinuous phase; and
   (v) optionally one or more auxiliary gel, lotion or cream forming components which may be present in the continuous and/or discontinuous phase;
(b) dispensing said personal care composition on and/or into the skin, hair and/or mucous membrane of the human being treated by discharging at least a portion of the personal care composition from said container into ambient pressure conditions to form a foam. For the purposes of convenience, a method according to the present invention is referred to herein as

### Method 2.

As used herein, the term "ambient pressure conditions" means at or about atmospheric pressure.

The present invention also includes methods of treating human skin, hair and/or mucous membranes comprising:
(a) providing a personal care composition in a container under pressure in the form of a stable oil-in-water emulsion comprising:
   (i) a continuous aqueous phase comprising water;
   (ii) a discontinuous phase comprising trans-1-chloro-3,3,3-trifluoropropene (1233zd(E)) and trans-1-3,3,3-tetrafluoropropene (1234ze(E)), wherein said 1233zd(E) and said 1234ze (E) together comprise from about 40% to about 60% by weight of said personal care composition and are present in a trans1233zd:trans1234ze weight ratio of from about 1:8 to less than 1:1;
   (iii) a solid emulsifier having an HLB value of from about 7 to about 18 and which may be present in the continuous and/or discontinuous phase; and
   (iv) from about 9% to less than about 14% by weight of a stability assistance component, preferably selected from the group consisting of ester oil, silicone, fatty alcohol, wax and combinations of two or more of these and which may be present in the continuous and/or discontinuous phase;
   (v) one or more active ingredients which may be present in the continuous and/or discontinuous phase; and
   (vi) optionally one or more auxiliary gel, lotion or cream forming components which may be present in the continuous and/or discontinuous phase; and
(b) dispensing said personal care composition on and/or into the skin, hair and/or mucous membrane of the human being treated by discharging at least a portion of the personal care composition from said container into ambient pressure conditions to form a foam. For the purposes of convenience, a method according to the present invention is referred to herein as

### Method 3.

As used herein, the term "stability assistance component" refers to those components, other than the emulsifier, which are described herein and which applicants have found are able to assist in achieving a stable oil-in-water emulsion for the formulation of the present invention.

The present invention also includes methods of treating human skin, hair and/or mucous membranes comprising:
(a) providing a personal care composition in a container under pressure in the form of a stable oil-in-water emulsion comprising:
   (i) a continuous aqueous phase comprising water;
   (ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise at least about 60% by weight of said personal care composition and are present in a trans1233zd:trans1234ze weight ratio of from about 1:8 to less than about 1:1; and
   (iii) an emulsifier having an HLB value of from about 7 to about 18 and which may be present in the continuous and/or discontinuous phase;
   v) one or more active ingredients which may be present in the continuous and/or discontinuous phase; and
   (vi) optionally one or more auxiliary gel, lotion or cream forming components which may be present in the continuous and/or discontinuous phase; and
(b) dispensing said personal care composition on and/or into the skin, hair and/or mucous membrane of the human being treated by discharging at least a portion of the personal care composition from said container into ambient pressure conditions to form a foam on the user and to provide the user with a cooling sensation. For the purposes of convenience, a method according to the present invention is referred to herein as **Method 4.**

The present invention also includes methods of treating human skin, hair and/or mucous membranes comprising:
(a) providing a personal care composition in a container under pressure in the form of a stable oil-in-water emulsion comprising:
   (i) a continuous aqueous phase comprising water;
   (ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise at least about 40% by weight of said personal care composition and are present in a trans1233zd:trans1234ze weight ratio of from about 1:8 to less than about 8:1; and
   (iii) a solid, long chain linear emulsifier in an amount effective to ensure that said oil-in-water emulsion is stable and to provide an emulsifier HLB of from about 7 to about 18, and which may be present in the continuous and/or discontinuous phase;
   v) one or more active ingredients which may be present in the continuous and/or discontinuous phase; and
   (vi) optionally one or more auxiliary gel, lotion or cream forming components which may be present in the continuous and/or discontinuous phase; and
(b) dispensing said personal care composition on and/or into the skin, hair and/or mucous membrane of the human being treated by discharging at least a portion of the personal care composition from said container into ambient pressure conditions to form a foam on the user in a form selected from sherbet foam, ice cream foam; crackling foam or a combination of two or more of these. For the purposes of convenience, a method according to the present invention is referred to herein as **Method 5.**

The present invention also includes methods of treating human skin, hair and/or mucous membranes comprising:
(a) providing a personal care composition in a container under pressure in the form of a stable oil-in-water emulsion comprising:
   (i) a continuous aqueous phase comprising water;
   (ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise at least about 40% by weight of said personal care composition and are present in a trans1233zd:trans1234ze weight ratio of from about 1:8 to less than about 8:1; and
   (iii) an emulsifier in an amount effective to ensure that said oil-in-water emulsion is stable and which may be present in the continuous and/or discontinuous phase;
   v) one or more active ingredients which may be present in the continuous and/or discontinuous phase; and
   (vi) optionally one or more auxiliary gel, lotion or cream forming components which may be present in the continuous and/or discontinuous phase; and
(b) dispensing said personal care composition on and/or into the skin, hair and/or mucous membrane of the human being treated by discharging at least a portion of the personal care composition from said container into ambient pressure conditions to form a foam on the user in a form selected from sherbet foam, ice cream foam; crackling foam or a combination of two or more of these. For the purposes of convenience, a method according to the present invention is referred to herein as **Method 6.**

As used herein, the term "sherbet foam" means a foam that is translucent and has a textured surface.

As used herein, the term "ice cream foam" means a foam that has a highly textured, non-smooth surface that contains numerous relatively sharp but soft points/ discontinuities.

The present invention also includes methods of treating human skin, hair and/or mucous membranes, including each of Methods 1 - 6, in which said step of forming foam comprises forming foam during delivery and/or while in contact with the skin, hair and/or mucous membrane by evaporating said trans-1-chloro-3,3,3-trifluoropropene (1233zd(E)) to form a foam that is in a form selected from sherbet foam, ice cream foam; crackling foam; or a combination of two or more of these.

The present invention also includes a personal care composition under pressure in a container for forming a personal care foam at ambient pressure wherein said foam is in a form selected from sherbet foam, ice cream foam; crackling foamor a combination of two or more of these, said composition comprising a substantially homogeneous oil-in-water emulsion comprising:
(a) a continuous aqueous phase comprising water;
(b) one or more personal care active components;
(c) from about 40% to about 60% by weight of a combination of trans 123 3zd and trans-1234ze in a trans1233zd:trans1234ze weight ratio of from 1:8 to 8:1; and
(d) one or more non-ionic emulsification agents, wherein said one or more non-ionic emulsification agents together have an HLB value of greater than 4,
wherein at least said transHFCO1233zd and transHFO-1234ze comprise one or more disperse phases in said continuous aqueous phase. For the purposes of convenience, a method according to the present invention is referred to herein as **Personal Care Composition 1.**

The present invention also includes a personal care composition under pressure in a container for forming a personal care foam at ambient pressure wherein said foam is in a form selected from sherbet foam, ice cream foam; crackling foam; or a combination of two or more of these, said composition comprising a substantially homogeneous oil-in-water emulsion comprising:
(a) a continuous aqueous phase comprising water, said continuous phase comprising at least about 40% by weight of the personal care composition;
(b) one or more personal care active components;
(c) from about 40% to about 60% by weight, based on the total weight of the personal care composition, of a combination of trans1233zd and trans-1234ze in a trans1233zd:trans1234ze weight ratio of from 1:8 to 8:1; and
(d) one or more liquid emulsification agents and one or more water-based thickeners, wherein said one or more emulsification agents together have an HLB value of greater than 4,
wherein at least said transHFCO1233zd and transHFO-1234ze comprise one or more disperse phases in said continuous aqueous phase. For the purposes of convenience, a method according to the present invention is referred to herein as **Personal Care Composition 2.**

The present invention also includes stable oil-in-water emulsions comprising:
(i) a continuous aqueous phase comprising water;
(ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise from about 40% to about 60% by weight of said personal care composition and are present in a trans1233zd:trans1234ze weight ratio of from about 8:1 to about 1:1;
(iii) an emulsifier having an HLB value of from about 7 to about 18 and which may be present in the continuous and/or discontinuous phase;
(iv) one or more active ingredients which may be present in the continuous and/or discontinuous phase; and
(v) optionally one or more auxiliary gel, lotion or cream forming components which may be present in the continuous and/or discontinuous phase. For the purposes of convenience, a method according to the present invention is referred to herein as

### Personal Care Composition 3.

The present invention also includes a personal care composition in the form of a stable oil-in-water emulsion comprising:
(i) a continuous aqueous phase comprising water;
(ii) a discontinuous phase comprising trans-1-chloro-3,3,3-trifluoropropene (1233zd(E)) and trans-1-3,3,3-tetrafluoropropene (1234ze(E)), wherein said 1233zd(E) and said 1234ze (E) together comprise from about 40% to about 60% by weight of said personal care composition and are present in a trans1233zd:trans1234ze weight ratio of from about 1:8 to less than 1:1;
(iii) a solid emulsifier having an HLB value of from about 7 to about 18 and which may be present in the continuous and/or discontinuous phase; and
(iv) from about 9% to less than about 14% by weight of a stability assistance component, preferably selected from the group consisting of ester oil, silicone, fatty alcohol, wax and combinations of two or more of these and which may be present in the continuous and/or discontinuous phase;
(v) one or more active ingredients which may be present in the continuous and/or discontinuous phase; and
(vi) optionally one or more auxiliary gel, lotion or cream forming components which may be present in the continuous and/or discontinuous phase. For the purposes of convenience, a method according to the present invention is referred to herein as

### Personal Care Composition 4.

The present invention also includes a personal care composition in the form of a stable oil-in-water emulsion comprising:
(i) a continuous aqueous phase comprising water;
(ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise at least about 60% by weight of said personal care composition and are present in a trans1233zd:trans1234ze weight ratio of from about 1:8 to less than about 1:1; and
(iii) an emulsifier having an HLB value of from about 7 to about 18 and which may be present in the continuous and/or discontinuous phase;
v) one or more active ingredients which may be present in the continuous and/or discontinuous phase; and
(vi) optionally one or more auxiliary gel, lotion or cream forming components which may be present in the continuous and/or discontinuous phase. For the purposes of convenience, a method according to the present invention is referred to herein as

### Personal Care Composition 5.

The present invention also includes a personal care composition in the form of a stable oil-in-water emulsion comprising:
(i) a continuous aqueous phase comprising water;
(ii) a discontinuous phase comprising foam forming component(s), trans-1-chloro-3,3,3-trifluoropropene (1233zd(E)) and trans- 1 -3,3,3 -tetrafluoropropene (1234ze(E)), wherein said 1233zd(E) and said 1234ze (E) together comprise at least about 40% by weight of said personal care composition and are present in a trans1233zd:trans1234ze weight ratio of from about 1:8 to less than about 8:1; and
(iii) an emulsifier in an amount effective to ensure that said oil-in-water emulsion is stable and which may be present in the continuous and/or discontinuous phase;
v) one or more active ingredients which may be present in the continuous and/or discontinuous phase; and
(vi) optionally one or more auxiliary gel, lotion or cream forming components which may be present in the continuous and/or discontinuous phase. For the purposes of convenience, a method according to the present invention is referred to herein as

### Personal Care Composition 6.

It will be appreciated by those skilled in the art that the ingredients that are used to form the gel, lotion or cream may themselves be considered to have an active function in the sense they provide a desired effect to the skin, hair or mucous membrane. By way of example, a skin conditioner may be in the form of a cream, which itself is considered to be an active ingredient of the formulation. Accordingly, the term "auxiliary gel, lotion or cream forming component" is used herein to identify ingredients which are included to in the formulation primarily for purposes other than improving the skin or hair, such as to impact the viscosity, feel, texture, delivery properties and the like of the formulation.

As used herein, the term "foaming said gel, lotion or cream" and similar terms mean that at least a substantial proportion of the gel, lotion, cream or the like is foamed. The term thus includes within its meaning steps in which some portion of the gel, lotion or cream remains unfoamed, although in preferred embodiments of the invention substantially all (e.g., 90% or more), of gel, lotion, cream or the like is foamed.

The present invention also includes methods of treating human skin, hair and/or mucous membranes, including each of Methods 1 - 6, in which the step of forming the foam comprises evaporating said trans-1-chloro-3,3,3-trifluoropropene and in which includes spreading said a gel, lotion, cream and/or foam on and/or into the skin, hair and/or mucous membrane of the human being treated.

The present invention also includes methods of treating human skin, hair and/or mucous membranes, including each of Methods 1 - 6, and personal care compositions, including each of Personal Care Compositions 1-6, wherein said foam forming component(s) consists essentially of or consist of 1233zd(E) and 1234ze(E).

The present invention also includes methods of treating human skin, hair and/or mucous membranes, including each of Methods 1, 2 and 4-6, and personal care compositions, including each of Personal Care Compositions 1-3 and 6 wherein said 1233zd(E) and said 1234ze (E) are present in a trans1233zd:trans1234ze weight ratio of from about 4:1 to about 1:1.

The present invention also includes methods of treating human skin, hair and/or mucous membranes, including each of Methods 1, 2 and 4-6, and personal care compositions, including each of Personal Care Compositions 1 - 3 and 6 wherein said 1233zd(E) and said 1234ze (E) are present in a trans1233zd:trans1234ze weight ratio of from about 2:1 to about 1:1.

The present invention also includes methods of treating human skin, hair and/or mucous membranes, including each of Methods 1, 2 and 4-6, and personal care compositions, including each of Personal Care Compositions 1 - 3 and 6 wherein said 1233zd(E) and said 1234ze (E) are present in a trans1233zd:trans1234ze weight ratio of from about 4:1 to about 1:1 and said foam is in the form of sherbet foam.

The present invention also includes methods of treating human skin, hair and/or mucous membranes, including each of Methods 1, 2 and 4-6, and personal care compositions, including each of Personal Care Compositions 1 - 3 and 6 wherein said 1233zd(E) and said 1234ze (E) are present in a trans1233zd:trans1234ze weight ratio of from about 4:1 to about 2:1 and said foam is in the form of sherbet foam.

The present invention also includes methods of treating human skin, hair and/or mucous membranes, including each of Methods 1 and 3-6, and personal care compositions, including each of Personal Care Compositions 1, 2, and 4 - 6 wherein said 1233zd(E) and said 1234ze (E) are present in a trans1233zd:trans1234ze weight ratio of from about 1:4 to less than 1:1.

The present invention also includes methods of treating human skin, hair and/or mucous membranes, including each of Methods 1 and 3 - 6, and personal care compositions, including each of Personal Care Compositions 1, 2, and 4 - 6 wherein said 1233zd(E) and said 1234ze (E) are present in a trans1233zd:trans1234ze weight ratio of from about 2:1 to less than about 1:1.

The present invention also includes methods of treating human skin, hair and/or mucous membranes, including each of Methods 1 and 3-6, and personal care compositions, including each of Personal Care Compositions 1, 2, and 4 - 6 wherein said 1233zd(E) and said 1234ze (E) are present in a trans1233zd:trans1234ze weight ratio of from about 1:4 to less than 1:1 and said foam is in the form of ice cream foam.

The present invention also includes methods of treating human skin, hair and/or mucous membranes, including each of Methods 1 and 3 - 6, and personal care compositions, including each of Personal Care Compositions 1, 2, and 4-6 wherein said 1233zd(E) and said 1234ze (E) are present in a trans1233zd:trans1234ze weight ratio of from about 1:4 to about 1:2 and said foam is in the form of ice cream foam.

The present invention includes methods of treating human skin, hair and/or mucous membranes comprising applying to the human skin, hair and/or mucous membranes a personal care composition of the present invention, including each of Personal Car Formulations 1-6.

The present invention includes methods of treating human skin, hair and/or mucous membranes comprising spreading on the human skin, hair and/or mucous membranes a personal care composition of the present invention, including each of Personal Car Formulations 1-6.

The present invention includes methods of treating human skin, hair and/or mucous membranes comprising delivering to the human skin, hair and/or mucous membranes a personal care composition of the present invention, including each of Personal Car Formulations 1-6, and then foaming the delivered personal care composition.

The present invention includes methods of treating human skin, hair and/or mucous membranes comprising delivering to the human skin, hair and/or mucous membranes a personal care composition of the present invention, including each of Personal Car Formulations 1 - 6, and then foaming the delivered personal care composition by evaporation of said form forming component

The present invention includes methods, including each of Methods 1- 6, of treating human skin, hair and/or mucous membranes comprising:(a) providing a personal care composition comprising one or more active ingredients and trans-1-chloro-3,3,3-trifluoropropene (1233zd(E)), where said one or more active ingredients are carried by and/or with said 1233zd(E).

As used herein, the term "evaporating said trans-1-chloro-3,3,3-trifluoropropene (1233zd(E))" and similar terms mean that at least a substantial proportion of the 1233zd is evaporated. The term thus includes within its meaning steps in which some portion of 1233zd remains unevaporated, although in preferred embodiments of the invention substantially all (e.g., 90% or more), of the 1233zd is evaporated. It will also be appreciated that the requirement that said one or more active ingredients remains on and/or in the skin, hair and/or mucous membrane of the human being treated contemplates both "stay on" compositions and methods and "rinse off" compositions and methods. Thus, the present invention includes methods in which the active ingredient is intended to remain on the skin, hair, and/or mucous membrane of the use without active intention or effort of the user to remove the product. In such "leave on" applications it is preferred that the substantially all of the 1233zd will be evaporated over a substantial period of time (e.g., 5 minutes or more) after application. On the other hand, in "rinse off" applications it is contemplated that an affirmative action will be taken to remove the cream, lotion, gel, foam or the like after application (such as by rinsing with water for example), and in such an embodiment it is contemplated that some portion, and potentially a substantial portion of the 1233zd will be removed from the user prior to evaporation. Thus, both "stay on" and "rinse off" applications are within the scope of the present invention.

Those skilled in the art will appreciate that the present invention includes articles that comprise a closed container which is openable to dispense the personal care composition of the present invention, including each of Personal Care Compositions 1 - 6. The invention thus includes containers in which the container is openable but not readily re-closed (such as would be the case of a sachet or the like which may be torn or ripped to provide an opening for dispensing the personal care formulation) and containers which are re-closable (such as would be the case of can or the like which has a nozzel with a valve that can be opened to allow dispensing of the personal care formulation and then re-closed to maintain the remaining portion of the formulation in the can for use at a later time.) In either case, but primarily in the case of a re-closable container, it is contemplated that the foaming component(s) of the present invention may also provide a force tending to expel the personal care formulation from the container. By way of example, in preferred embodiments in which the foaming component comprises both 1233zd and 1234ze, the 1234ze component of the personal care composition will generally exit the container with the formulation and serve not only to provide a foaming action but will also serve to at least assist in expelling the personal care formulation from the container. In such embodiments, the formulation preferably includes both the 1233zd(E) and the 1234ze(E) as components that are miscible with and/or dispersed in and/or emulsified with the formulation, and in such a case, the 1234ze(E) component would contribute to foaming of the formulation as it exits the container as well as providing a propelling force. For the purposes of the description of the present invention, 1234ze and 1233zd in such embodiments, and other components which behave in the same or similar manner, are within the term "foaming component(s)" and the like since it contributes substantially to foaming of the personal care composition.

### DETAILED DESCRIPTION

The preferred methods, articles and compositions of the present invention provide one or more, and preferably several, unexpected performance properties compared to prior personal care methods and personal care compositons. These performance advantages include:
- improved and enhanced sensations to the user, including appearance, feel and sound;
- improved stability of the emulsion that forms the foam
- improved penetration of the active ingredient into the skin, hair and/or mucous membrane of the user
- cooling sensation to the skin/mucous membrane that is or at least is perceived to be felt essentially immediately upon application
- enhanced and long lasting cooling sensation provided to the user, preferably without the use of sensates.

As used herein, the terms "personal care composition," personal care formulation" and the like means compositions, formulations and the like that are to be topically applied to a person.

The personal care compositions and formulations of the present invention can be in the form, for example, of lotions, creams, gels and the like, and in each of such forms the composition is preferably a post-foaming composition or formulation. It will be appreciated, therefore, that while post-foaming formulation are preferred in many applications, lotions, creams, gels and the like that are not post-foaming are also within the broad scope of the present invention, including particularly embodiments which provide cooling sensation to the user. The compositions and formulations of the present invention can be in the form of sprayed or aerosolized powders, mists and the like which generally are not post-foaming compositions but which provide the advantageous cooling sensation of the present invention.

As used herein, the term "post-foaming" refers to gels, creams, lotions and the like which as applied are essentially not in the form of a foam but which as a result of the application of heat (which may be in the form of body heat) or other energy (such as kinetic energy associated with rubbing or spreading the formulation on the skin and/or in the hair, for example) produce a foamed product. For the purposes of convenience, but not necessarily by way of limitation, compositions, formulations and methods associated with post-foaming action are described below separately from non-post foaming compositions, formulations and methods.

### Active Components

The active component(s) used in the compositions and methods of the present invention can have a wide variety of purposes and functions, and all such components are within the scope of the present invention. In general, such components will provide or at least be expected to provide some benefit to the user, preferably to the skin, hair and/or mucous membrane of the user. Thus, the active components can include cosmetic agents (that is, agents that are cosmetically treating, providing nutrition to and/or conditioning the skin, hair or mucous membrane via topical application), medicinal agents (that is, agents that capable of promoting or assisting in the recovery from injury or illness, preferably to the skin, hair or mucous membrane), and pharmaceutical agents (that is, drugs that are appropriate for delivery by topical application, including preferably drugs that are effective at treating skin, hair and mucous membranes). It will be appreciate by those skilled in the art that active ingredients can have more than one mode of operation and/or effect and therefore may be properly included in two or more categories of product for the purposes of the present invention.

Examples of active components include but are not limited to: coloring, decoloring and appearance altering agents (such as pigments, depigmentation agents, skin tighteners, reflectants and the like); hair treatment agents (such as shaping agents, detangling agents, wet combing agents, anti-hair loss agents, hair growth promoting agents, hair growth inhibitors, hair colorants, hair conditioners, hair softeners; hair moisturizers, anti-dandruff agents and the like); film forming polymers; humectants; amino acids and their derivatives; antimicrobial agents; allergy inhibitors; anti-acne agents (such as salicylic acid, benzoyl peroxide, sulfur, adapalene and glycolic acid); anti-aging and anti-wrinkling agents (such as dimethylaminoethanol ("DMAE"), retinol, Vitamin C, hydroxy acids, perptides, tea extracts, grape seed extracts and niacinamide), antiseptics; analgesics; antitussives; anti-pruritics; local anesthetics; antihistamines; anti-infective; inflammation inhibitors; deodorants and anti-perspirants (aluminium chlorohydrate and aluminium-zirconium tetrachlorohydrate); medicament agents; skin emollients and skin moisturizers; skin firming agents; anti-fungal agents; shaving preparations, lotions, creams and gels; perfumes; make-up preparations; cosmetics (such as skin cleansers, skin conditioners, make-up and the like); and sun screen and sun blocks (such as benzophenones, bomelone, butyl paba, cinnamidopropyl trimethyl ammonium chloride, disodium distyrylbiphenyl disulfonate, paba, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate 0, and red petrolatum); insect repellants, skin protectants (such as atmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like), vitamins (such as vitamin B complex; including thiamine, nicotinic acid, biotin, pantothenic acid, choline, riboflavin, vitamin B6, vitamin B12, pyridoxine, inositol, carnitine; vitamins A,C,D,E,K and their derivatives such as vitamin A palmitate and pro-vitamins, e.g. (i.e. panthenol (pro vitamin B5) and panthenol triacetate).

The amount of the active component(s) to be used with the present invention can vary widely depending on the particular application, and all such amounts can be determined by those skilled in the art in view of the teachings contained herein and are within the broad scope of the invention. For example, amount of the active component(s) may vary depending upon the ability of the component to penetrate through, onto, or into the pores of the skin, hair or mucous membrane, in view of the enhancements thereto provided by the present invention. Other factors which may influence the relative amount of the active component include the particular active component involved, the particular benefit desired, the sensitivity of the user to the active component, the health condition, age, and skin, hair, and/or mucous membrane condition of the user, and the like. In sum, the benefit agent is preferably used in a "safe and effective amount," which is an amount that is high enough to deliver a desired skin, hair or mucous membrane benefit or to modify a certain condition to be treated and which avoids unwanted deleterious serious side effects. In preferred embodiments, the active components together are present in the personal care composition or personal care formulation system in an amount, based upon the total weight of the composition/formulation, of from about 0.01 percent to about 50 percent by weight, or from about 0.01 percent to about 20 percent, or from about 0.01 percent to about 10 percent or from about 0.01 percent to about 5 percent, or from about 0.01 percent to about 1.0 percent by weight.

### Auxiliary Gel, Lotion Or Cream Forming Components

The types of auxiliary components that may be used in the formulations of the present invention can vary widely depending on the particular form of the formulation (e.g., gel, cream or lotion) and the particular attributes of the formulation desired, and any and all such components are included within the scope of the present invention.

In connection with gels, for example, the gel forming component in preferred embodiments comprises, preferably in major proportion based on the total weight of the formulation, an aqueous soap. The soap in the aqueous soap component can be, for example, either a soap or a surface active agent, which is also sometimes called a wetting agent, which is generally anionic or nonionic in character.

The soaps (water soluble salts of higher fatty acids) which are preferably amine or basic salts, that suitable for the production of a post-foaming gel are generally known in the art, and all such soaps are within the scope of the present invention. In general, such a component can preferably be an alkali, ammonium or soluble amine salt of a fatty acid such as stearic acid, palmitic acid, myristic acid and the like. As known in the art, soaps may also be prepared by neutralization or saponification of animal fats, such as tallow or vegetable fats. The selection of a suitable soap component is deemed to be within the scope of those skilled in the art from the teachings herein.

The anionic or nonionic surface active agent which can be employed instead of a soap, or in conjunction therewith, should be one which is appreciably soluble in water, and as examples of such agents there may be mentioned triethanolamine lauryl sulfate, sodium lauryl sulfate, sodium dodecyl benzene sulfonate, water-soluble polyoxyethylene ethers of alkyl-substituted phenols and water-soluble polyoxyethylene cetyl ethers. The selection of a suitable wetting agent is deemed to be within the scope of those skilled in the art from the teachings herein.

Preferably in the present methods, including each of Methods 1-6, and in the present personal care compositions, including each of Personal Care Compositions 1 - 6, the emulsifier comprises or consists essentially of a non-ionic, solid, long chain, linear emulsifier having an HLB value of from about 9 to about 16. One example of such an emulsifier is Tween 20.

It has been acknowledged that one difficulty associated with the formation of gels for use in post-foaming applications is the difficulty of forming a gel that in its unfoamed state is not easily spreadable over the skin or hair in an even and fluid manner. See, for example, US 5,334,325, which describes the use of particular alkoxylated alkyl phosphate ester surfactant surfactants, the contents of which are incorporated herein by reference.

Other auxiliary ingredients that may be included in the present gels, creams and lotions for use in post-foaming applications include polymers for gel thickening and lubrication, cosmetic esters for emolliency, humectants for moisturization, fragrances, dyes and preservatives.

The amount of the auxiliary component(s) to be used with the present invention can vary widely depending on the particular application, and all such amounts can be determined by those skilled in the art in view of the teachings contained herein and are within the broad scope of the invention. In preferred embodiments, the auxiliary component(s) together are present in the personal care composition or personal care formulation system in an amount, based upon the total weight of the composition/formulation, of from about 1 percent to about 60 percent based on the total weight of the components in formulation.

As mentioned above, the present invention also includes creams, lotions, gels, foams, mists, powders, sprays and the like which do not exhibit post-foaming action. By way of example, the advantageous cooling effect as described herein can be provided for even creams, lotions, gels, foams, mists, powders, sprays and the like which are designed to not exhibit an post-foaming action but which nevertheless in which the 1233zd of the present invention evaporates after the creams, lotions, gels, foams, mists, powders, sprays and the like are applied to the skin, hair and/or mucous membrane of the user and thus provide the advantageous cooling effect as described herein. By way of further example, the advantageous ability of the present formulations, by virtue of inclusion in part of the 1233zd of the present invention, provides an advantageous ability of the active ingredient to penetrate into and/or be delivered directly into the skin/mucous membrane pores of the user and/or the hair follicles of the user even in embodiments in which post-foaming action is not achieved or desired.

Accordingly, the non-post-foaming embodiments of the present invention, can also utilize formulations and compositions in the form of creams, lotions, gels, foams, mists, powders, sprays and the like in which the formulations and compositions generally comprise: (a) one or more active components, preferably comprising a cleanser; (b) optionally but preferably one or more auxiliary components which help to form the creams, lotions, gels, foams, mists, powders, sprays and the like; (c) a foaming agent comprising, or consisting essentially of, or consisting of 1233zd(E) and 1234ze(E); and (d) optional additional components other than components (a), (b) and (c) that may be included in the composition. The types and amounts of components (a), (b) and (c) in such non-fost-foaming applications can vary broadly and include the types and amounts of such components as described herein in connection with post-foaming applications. In the non-post-foaming embodiments, for the purpose of convenience, but not by way of limitation, the components (a), (b) and (d) are also sometimes referred to herein as the "formulation base," or the "cleanser base" (in the case where the active component includes a cleanser) and the like.

The present personal care compositions can be in the form of and/or used in a wide variety of personal care products/applications, including: shampoos (including baby shampoos, conditioning shampoos, moisturizing shampoos, etc.); skin and body cleansers (including shower gels, bath foams, facial cleansers, intimate cleansers, moisturizing body washes, antibacterial body washes; bath gels; hand soaps; facial scrubs; foot scrubs, etc); hair care compositions (including hair dyes, hair conditioners, hair creams and hair styling formulations); skin care compositions (including skin lotions, balms and creams, skin whiteners, self tanning lotions, sunscreen lotions, carrier lotions, moisturizers, vitamin C creams, antibacterial lotions, facial masks, body masks, make up foundations, sun care formulations, antiperspirants, etc).

### EXAMPLES Example 1A - Hydrating Body Lotion to Form Ice Cream Foam

A hydrating body lotion was prepared using a hydrating base comprising th following components: water, Cetearyl Alcohol (as a stability assistance component), Glycerin, Cetyl Alcohol(as a stability assistance component), Behentrimonium Chloride (as a cationic emulsifier), Argania Spinosa (Argan) Kernel Oil, Silk Amino Acids, Dimethicone, Cyclopentasiloxane (stability assistance component, Dimethiconol, Glycol Distearate (as a stability assistance component with an HLB of 1), Glycol Stearate (stability assistance component with an HLB of 2.9), Ceteareth-20 (as an emulsifier with an HLB of 15.2), Isopropyl Alcohol, Citric Acid, Phenoxyethanol, Ethylhexylglycerin, DMDM Hydantoin, Fragrance (Parfum), Red 40 (CI 16035), Yellow 5 (CI 19140), Yellow 6 (CI 15985Water). Several formulations using this hydrating base and various amounts of trans1233zd, trans1234zd in relative amounts indicated in Table 1 below, with all amounts being in percentage by weight:

**Table 1 - Hydrating Body Lotion**

| **Ingredient** | **Dosage Formulations** | | | | | |
|---|---|---|---|---|---|---|
| | **A** | **B** | **C** | **D** | **E** | **F** |
| hydrating base | **50** | **60** | **70** | **80** | **80** | **70** |
| 1233zd | **10** | **8** | **6** | **4** | | |
| 1234ze | **40** | **32** | **24** | **16** | **20** | **30** |
| Total | **100** | **100** | **100** | **100** | **100** | **100** |
| Wt ratio 1233zd/1234ze | **1:4** | **1:4** | **1:4** | **1:4** | **NA** | **NA** |

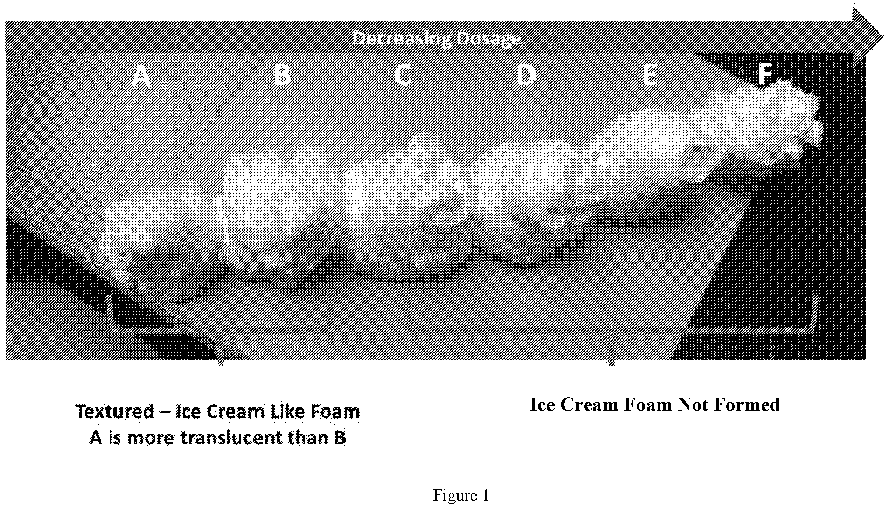

As can be seen from Figure 1, Formulation A and Formulations B, which each utilized a total amount of trans 123 3zd plus trans1234ze of 40% or greater and a zd/ze weight ratio of 1:4 formed textured, ice-cream like foam and which had a translucent character. In contrast, Formulations C and D produced a foam with a relatively smooth continuous surface and relatively little or no translucent character. In these cases, even though the zd/ze weight ratio was 1:4, the total dossage was less than 40% (30% and 20%, respectively).

### Example 1B - Hydrating Body Lotion to Form Ice Cream Foam

Example 1A is repeated except that zd/ze weight ratio for formulations A and B is adjusted to 1:1 (i.e., 25wt% zd and 25wt% ze for Formulation A and 20wt% zd and 20 wt% ze for formulation B). Similar results are obtained.

### Example 1C - Hydrating Body Lotion to Form Ice Cream Foam

Example 1A is repeated except that zd/ze weight ratio for formulations A and B is adjusted to 1:2 (i.e., 16.67 wt% zd and 33.33 wt% ze for Formulation A and 1.3.3wt% zd and 26.7 wt% ze for formulation B). Similar results are obtained.

### Example 2A - Hydrating Body Lotion to Form Sherbet Foam

A hydrating body lotion was prepared using a hydrating base comprising th following components: water (Aqua), Cetearyl Alcohol (as a stability assistance component), Glycerin, Cetyl Alcohol(as a stability assistance component), Behentrimonium Chloride (as a cationic emulsifier), Argania Spinosa (Argan) Kernel Oil, Silk Amino Acids, Dimethicone, Cyclopentasiloxane (stability assistance component), Dimethiconol, Glycol Distearate (as a stability assistance component with an HLB of 1), Glycol Stearate (as a stability assistance componentwith an HLB of 2.9), Ceteareth-20 (as an emulsifier with an HLB of 15.2), Isopropyl Alcohol, Citric Acid, Phenoxyethanol, Ethylhexylglycerin, DMDM Hydantoin, Fragrance (Parfum), Red 40 (CI 16035), Yellow 5 (CI 19140), Yellow 6 (CI 15985Water). Several formulations using this hydrating base and various amounts of trans1233zd, trans1234zd in relative amounts indicated in Table 2 below, with all amounts being in percentage by weight:

**Table 2 - Hydrating Body Lotion**

| **Ingredient** | **Dosage Formulations** | | | |
|---|---|---|---|---|
| | **A** | **B** | **C** | **D** |
| hydrating base | **50** | **60** | **70** | **80** |
| 1233zd | **40** | **32** | **24** | **16** |
| 1234ze | **10** | **8** | **6** | **4** |
| Total | **100** | **100** | **100** | **100** |
| Wt ratio 1233zd/1234ze | **4:1** | **4:1** | **4:1** | **4:1** |

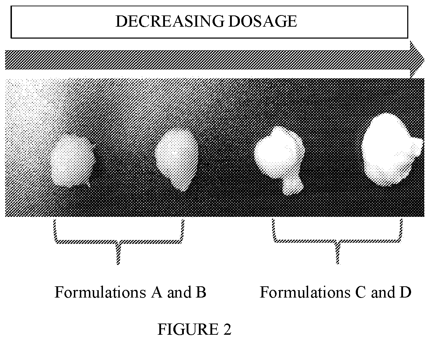

As can be seen from Figure 2, Formulation A and Formulations B, which each utilized a total amount of trans1233zd plus trans1234ze of 40% or greater and a zd/ze weight ratio of 4:1 formed a sherbet foam having a translucent character. In contrast, Formulations C and D produced a foam with a relatively smooth, continuous and opaque surface. In these cases, even though the zd/ze weight ratio was 4:1 the total dossage was less than 40% (30% and 20%, respectively).

### Example 2B - Hydrating Body Lotion to Form Sherbet Foam

Example 2A is repeated except that zd/ze weight ratio for formulations A and B is adjusted to 1:1 (i.e., 25wt% zd and 25wt% ze for Formulation A and 20wt% zd and 20 wt% ze for formulation B). Similar results are obtained.

### Example 2C - Hydrating Body Lotion to Form Sherbet Foam

Example 2A is repeated except that zd/ze weight ratio for formulations A and B is adjusted to 2:1 (i.e., 33.33t% zd and 16.67 wt% ze for Formulation A and 26.7 wt% zd and 13.3wt% ze for formulation B). Similar results are obtained.

Examples 1 and 2 thus reveal that that formulations in which trans1233zd and trans1234ze together comprise at least about 40% by weight of the personal care composition is preferred to form ice cream and sherbret type of foam .

Also, it is revealed that ratio of trans1233zd:trans1234ze of from about 4:1 to about 1:4 is preferred to form a sherbet like structure which also preferably crackles upon application and/or provides cooling upon application.

### Comparative Example 1 - Personal Care Formulation with Low HLB Emulsifier

A personal care formulation which contained 50% by weight of trans1233zd plus trans1234ze as depicted in Table C3 below is formed:

| **Ingredient** | **Function** | **Wt% Component** |
|---|---|---|
| | | **A** |
| water | **Solvent** | **47.5** |
| Glycerin | **humectant** | **1** |
| Carbopol 904 | **Thickener (water based)** | **0.25** |
| TEA | **Neutralizing Agent** | **0.25** |
| Euxyl K940 | **Preservative** | **0.25** |
| Brij 93 (HLB=4) | **Emulsifier (solid)** | **1** |
| 1233zd | | **10** |
| 1234ze | | **40** |
| Total | | **100** |

As can be seen from the Table above, the HLB of the emulsifier used in the formulation was Brij 93, which provided the formulation with emulsifiers having an HLB value of about 4. A stable emulsion was not formed, as measured by whether phase separation is observed when shaken 24 hours after formation at room temperature. Unless otherwise indicated herein, the test described herein was used in the examples hereof to determine the stability of the formulation, with the observance of phase separation indicating that the formulation is not stable and will not be acceptable for forming a foam,

### Example 3A, 3B, 3C and 3D - Personal Care Formulation with High HLB Emulsifier

A series of personal care formulations (3A, 3B, 3C and 3D) which contained 50% by weight of trans1233zd plus trans1234ze in a weight ratio of 1:4 as depicted in Table 3 below were formed:

**Table 3**

| **Ingredient** | **Function** | **Wt% Component** | | | |
|---|---|---|---|---|---|
| | | **3A** | **3B** | **3C** | **3D** |
| water | **Solvent** | 47.5 | 47.5 | 47.5 | 47.5 |
| Glycerin | **humectant** | 1 | 1 | 1 | 1 |
| Carbopol 904 | **Thickener (water based)** | 0.25 | 0.25 | 0.25 | 0.25 |
| TEA | **Neutralizing Agent** | 0.25 | 0.25 | 0.25 | 0.25 |
| Euxyl K940 | **Preservative** | 0.25 | 0.25 | 0.25 | 0.25 |
| Tween-20 (HLB=16.7) | Emulsifier (solid) | 0.4 | 0.65 | 0.8 | 1 |
| Brij 93 (HLB=4) | Emulsifier (solid) | 0.6 | 0.35 | 0.2 | 0 |
| 1233zd | | 10 | 10 | 10 | 10 |
| 1234ze | | 40 | 40 | 40 | 40 |
| Total | | 100 | 100 | 100 | 100 |
| HLB of Emulsifiers in Formulation | | 9.08 | 12.26 | 14.16 | 16.7 |

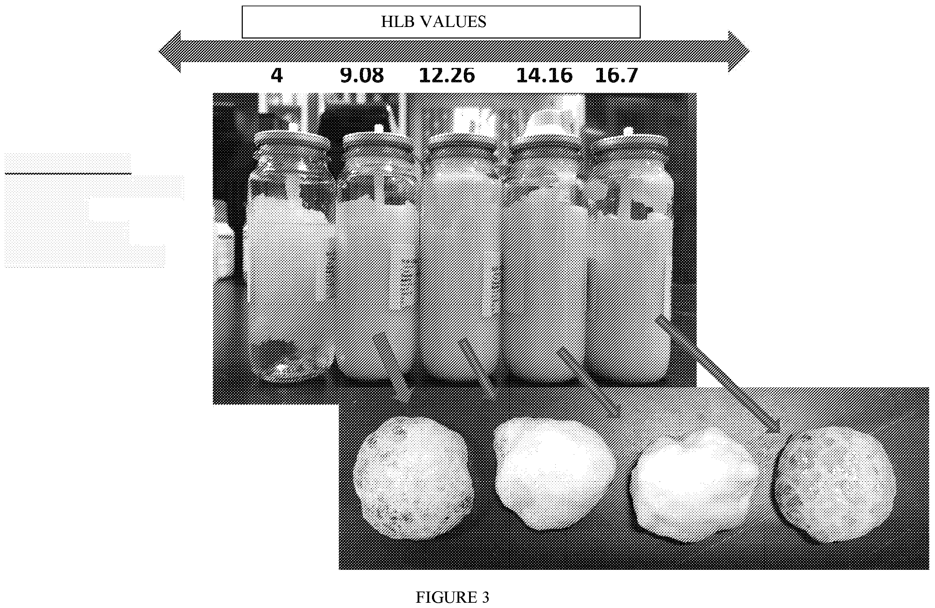

As can be seen from the table above, the series of personal care formulations had emulsifier HLB values of 9.08, 12.26, 14.16 and 16.7, for Examples 3A, 3B, 3C and 3D, respectively using a solid, long chain substantially linear non-ionic surfactant (Tween 20) to increase the HLB value of the emulsification package. A stable emulsion was formed in each case, and cooling foams were formed from each formulation. These results are revealed in Figure 3 hereof, with the photographs being from Comparative Example 1 and Examples 3A - 3D from left to right in the Figure. However, ice cream foam was not formed from any of the formulations.

### Comparative Examples 2A, 2B, 2C, 2D and 2E - Unstable Personal Care Formulations

A series of personal care formulation generally as described in Comparative Example 1 and Examples 3A - 3D which contained 50% by weight of trans1233zd plus trans1234ze, but without using a long chain substantially linear non-ionic surfactant to increase HLB as described in Table 4 below were formed:

**Table 4**

| **Ingredient** | **Function** | **Wt% Component** | | | | | |
|---|---|---|---|---|---|---|---|
| | | **C2A** | **C2B** | **C2C** | **C2D** | **C2E** | **C2F** |
| water | **Solvent** | 47.5 | 47.5 | 47.5 | 47.5 | 47.5 | 47.5 |
| Glycerin | **humectant** | 1 | 1 | 1 | 1 | 1 | 1 |
| Carbopol 904 | **Thickener (water based)** | 0.25 | 0.25 | 0.25 | 0.25 | 0.25 | 0.25 |
| TEA | **Neutralizing Agent** | 0.25 | 0.25 | 0.25 | 0.25 | 0.25 | 0.25 |
| Euxyl K940 | **Preservative** | 0.25 | 0.25 | 0.25 | 0.25 | 0.25 | 0.25 |
| Steareth-21 (ΣiI.B=15.55) | Emulsifier (solid) | 0 | 0.2 | 0.45 | 0.7 | 0.9 | 0.9 |
| Glyceryl Sterarate | Emulsifier (solid) | 1 | 0.8 | 0.55 | 0.3 | 0.1 | 0 |
| 1233zd | | 10 | 10 | 10 | 10 | 10 | 10 |
| 1234ze | | 40 | 40 | 40 | 40 | 40 | 40 |
| Total | | 100 | 100 | 100 | 100 | 100 | 100 |
| HLB of Emulsifiers in Formulation | | 3.8 | 6.14 | 9.06 | 12 | 14.33 | 15.55 |

None of the formulation as described in Table 4 formed a stable emulsion.

### Example 4A and 4B - Personal Care Formulation with High HLB liquid Emulsifier and Ester Oil

Two personal care formulations (4A and 4B) similar to Formulas C2C and C2D, but with the addition of an ester oil, were formed as described in Table 4 below:

**Table 4**

| **Ingredient** | **Function** | **Wt% Component** | |
|---|---|---|---|
| | | **4A** | **4B** |
| water | **Solvent** | 45.25 | 45.25 |
| Glycerin | **humectant** | 1 | 1 |
| Carbopol 904 | **Thickener (water based)** | 0.25 | 0.25 |
| TEA | **Neutralizing Agent** | 0.25 | 0.25 |
| Euxyl K940 | **Preservative** | 0.25 | 0.25 |
| Steareth-21 (ΣiI.B=15.55) | Emulsifier (solid) | 0.45 | 0.9 |
| Glyceryl Sterarate | Emulsifier (solid) | 0.55 | 0.1 |
| Isopropyl myristate | Stability assistance component | 2 | 2 |
| 1233zd | | 10 | 10 |
| 1234ze | | 40 | 40 |
| Total | | 100 | 100 |
| HLB of Emulsifiers in Formulation | | 9.06 | 14.33 |
| Stability of the formlation | | Stable | Stable |

As can be seen from the table above, the addition of 2% by weigh to ester oil i(sopropyl myristate) assisted with and enabled the creation of a stable personal care composition. However, ice cream foam was not formed from either of the formulations.

### Example 5A, 5B and 5C - Personal Care Formulation with High HLB liquid Emulsifier and Fatty Aclohol

Three personal care formulations (5A, 5B and 5C) similar to Formulas C2C but with the use of a liquid emulsifier with an HLB of 14 and the addition of 4 %, 8% and 12% of fatty alcohol were formed. The formulations that used 12% and 8% fatty alcohol did not form a stable emulsion, but the formulation that used the 4% of fatty alcohol formed a stable personal care composition, but an ice cream foam was not formed.

### Example 6A, 6B and 6C - Personal Care Formulation with High HLB solid Emulsifier, Fatty Aclohol and Ester Oil

Three personal care formulations (6A, 6B and 6C) similar to Formula C2C but with the use of a liquid emulsifier with an HLB of 14 and 4% ester oil and the addition of 4 %, 8% and 12%, respectively of fatty alcohol, were formed. All three formulations formed a stable emulsion, but an ice cream foam was not formed.

### Example 7A, 7B and 7C - Personal Care Formulations with High HLB solid Emulsifier, Fatty Aclohol and Ester Oil

Three personal care formulations (7A, 7B and 7C) similar to Formula C2C but with the use of a solid emulsifier with an HLB of 14 and 4% ester oil and the addition of 4 %, 8% and 12%, respectively of fatty alcohol, were formed. All three formulations formed a stable emulsion, but an ice cream foam was not formed.

### Example 8 - Personal Care Formulations with High HLB solid Emulsifier, Fatty Aclohol, Ester Oil and Silicone

A personal care formulations similar to Formula C2C but with the use of a solid emulsifier with an HLB of 15.5 (Steareth 21), 4% ester oil, 8% fatty alcohol, no water based thickener and 2% silicone were formed. The formulation formed a stable emulsion and produced an ice cream foam.

### Example 9A - Personal Care Formulations with High HLB solid Emulsifier, Fatty Aclohol, Ester Oil and Silicone

A personal care formulations similar to Formula C2C but with the use of a solid emulsifier with an HLB of 15.5 (Steareth 21), 2% ester oil, 12% fatty alcohol, no water based thickener and 2% silicone were formed. The formulation formed a stable emulsion and but did not produce ice cream foam.

### Example 9B - Personal Care Formulations with High HLB solid Emulsifier, Fatty Aclohol, Ester Oil, Silicone and Carnauba Wax

A personal care formulations similar to Formula C2C but with the use of a solid emulsifier with an HLB of 15.5 (Steareth 21), 2% ester oil, 4% fatty alcohol, no water based thickener, 2% silicone and 4% of carnauba wax were formed. The formulation formed a stable emulsion and produced an ice cream foam.

### Comparative Example 3 - Personal Care Formulation with Low HLB Emulsifier

A personal care formulation which contained 50% by weight of trans1233zd plus trans1234ze as depicted in Table C4 below is formed:

**TABLE C4**

| **Ingredient** | **Function** | **Wt% Component** |
|---|---|---|
| | | |
| water | **Solvent** | **47.5** |
| Glycerin | **humectant** | **1** |
| Carbopol 904 | **Thickener (water based)** | **0.25** |
| TEA | **Neutralizing Agent** | **0.25** |
| | | |
| Euxyl K940 | **Preservative** | **0.25** |
| Brij 93 (HLB=4) | **Emulsifier (solid)** | **1** |
| 1233zd | | **40** |
| 1234ze | | **10** |
| Total | | **100** |

As can be seen from the Table above, the HLB of the emulsifier used in the formulation was Brij 93, which provided the formulation with emulsifiers having an HLB value of about 4. A stable emulsion was not formed.

### Examples 10A, 10B, 10C and 10D - Personal Care Formulation with High Solid HLB Emulsifier

A series of personal care formulations (10A, 10B, 10C and 10D) which contained 50% by weight of trans1233zd plus trans1234ze in a weight ratio of 4:1 as depicted in Table 5 below were formed:

**Table 5**

| **Ingredient** | **Function** | **Wt% Component** | | | |
|---|---|---|---|---|---|
| | | **10A** | **10B** | **10C** | **10D** |
| water | **Solvent** | 47.5 | 47.5 | 47.5 | 47.5 |
| Glycerin | **humectant** | 1 | 1 | 1 | 1 |
| Carbopol 904 | **Thickener (water based)** | 0.25 | 0.25 | 0.25 | 0.25 |
| TEA | **Neutralizing Agent** | 0.25 | 0.25 | 0.25 | 0.25 |
| Euxyl K940 | **Preservative** | 0.25 | 0.25 | 0.25 | 0.25 |
| Tween-20 (HLB=16.7) | Emulsifier (solid) | 0.4 | 0.65 | 0.8 | 1 |
| Brij 93 (HLB=4) | Emulsifier (solid) | 0.6 | 0.35 | 0.2 | 0 |
| 1233zd | | 40 | 40 | 40 | 40 |
| 1234ze | | 10 | 10 | 10 | 10 |
| Total | | 100 | 100 | 100 | 100 |
| HLB of Emulsifiers in Formulation | | 9.08 | 12.26 | 14.16 | 16.7 |

As can be seen from the table above, the series of personal care formulations had emulsifier HLB values of 9.08, 12.26, 14.16 and 16.7, for Examples 10A, 10B, 10C and 10D, respectively using a solid, long chain substantially linear non-ionic surfactant (Tween 20) to increase the HLB value of the emulsification package. A stable emulsion was formed in each case, and sherbret foams that had a pleasant crackling sound were formed from each formulation.

### Comparative Examples 4A, 4B, 4C, 4D and 4E - Unstable Personal Care Formulations

A series of personal care formulation generally as described in Comparative Example 3 and Examples 10A - 10D which contained 50% by weight of trans1233zd plus trans1234ze, but without using a long chain substantially linear non-ionic surfactant to increase HLB as described in Table 4 below were formed:

**Table 4**

| **Ingredient** | **Function** | **Wt% Component** | | | | | |
|---|---|---|---|---|---|---|---|
| | | **C4A** | **C4B** | **C4C** | **C4D** | **C4E** | **C4F** |
| water | **Solvent** | 47.5 | 47.5 | 47.5 | 47.5 | 47.5 | 47.5 |
| Glycerin | **humectant** | 1 | 1 | 1 | 1 | 1 | 1 |
| Carbopol 904 | **Thickener (water based)** | 0.25 | 0.25 | 0.25 | 0.25 | 0.25 | 0.25 |
| TEA | **Neutralizing Agent** | 0.25 | 0.25 | 0.25 | 0.25 | 0.25 | 0.25 |
| Euxyl K940 | **Preservative** | 0.25 | 0.25 | 0.25 | 0.25 | 0.25 | 0.25 |
| Steareth-21 (ΣiI.B=15.55) | Emulsifier (solid) | 0 | 0.2 | 0.45 | 0.7 | 0.9 | 0.9 |
| Glyceryl Sterarate (HLB=3.8) | Emulsifier (solid) | 1 | 0.8 | 0.55 | 0.3 | 0.1 | 0 |
| 1233zd | | 40 | 40 | 40 | 40 | 40 | 40 |
| 1234ze | | 10 | 10 | 10 | 10 | 10 | 10 |
| Total | | 100 | 100 | 100 | 100 | 100 | 100 |
| HLB of Emulsifiers in Formulation | | 3.8 | 6.14 | 9.06 | 12 | 14.33 | 15.55 |

None of the formulation as described in Table 4 formed a stable emulsion.

### Example 11A and 11B - Personal Care Formulation with High HLB liquid Emulsifier and Ester Oil

Two personal care formulations (4A and 4B) similar to Formulas C2C and C2D, but with the addition of an ester oil, were formed as described in Table 5 below:

**Table 5**

| **Ingredient** | **Function** | **Wt% Component** | |
|---|---|---|---|
| | | **4A** | **4B** |
| water | **Solvent** | 45.25 | 45.25 |
| Glycerin | **humectant** | 1 | 1 |
| Carbopol 904 | **Thickener (water based)** | 0.25 | 0.25 |
| TEA | **Neutralizing Agent** | 0.25 | 0.25 |
| Euxyl K940 | **Preservative** | 0.25 | 0.25 |
| Steareth-21 (ΣiI.B=15.55) | Emulsifier (solid) | 0.45 | 0.9 |
| Glyceryl Sterarate | Emulsifier (solid) | 0.55 | 0.1 |
| Isopropyl myristate | Stability assistance component | 2 | 2 |
| 1233zd | | 40 | 40 |
| 1234ze | | 10 | 10 |
| Total | | 100 | 100 |
| HLB of Emulsifiers in Formulation | | 9.06 | 14.33 |
| Stability of the formlation | | Stable | Stable |

As can be seen from the table above, the addition of 2% by weigh to ester oil (isopropyl myristate) assisted with and enabled the creation of a stable personal care composition, and in addition a sherbet foam with a pleasant cracking sound was formed.

### Example 12A, 12B and 12C - Personal Care Formulation with High HLB liquid Emulsifier and Fatty Aclohol

Three personal care formulations (12A, 12B and 12C) similar to Formulas C4C but with the use of a liquid emulsifier with an HLB of 14 and the addition of 4 %, 8% and 12% of fatty alcohol were formed. The formulations that used 12% and 8% fatty alcohol did not form a stable emulsion, but the formulation that used the 4% of fatty alcohol formed a stable personal care composition, but an sherbet foam was not formed.

### Example 13A, 13B and 13C - Personal Care Formulation with High HLB solid Emulsifier, Fatty Aclohol and Ester Oil

Three personal care formulations (13A, 13B and 13C) similar to Formula C4C but with the use of a liquid emulsifier with an HLB of 14 and 4% ester oil and the addition of 4 %, 8% and 12%, respectively of fatty alcohol, were formed. All three formulations formed a stable emulsion, but a sherbet foam was not formed, although each foam exhibited a pleasant cracking sound.

### Example 14A, 14B and 14C - Personal Care Formulations with High HLB solid Emulsifier, Fatty Aclohol and Ester Oil

Three personal care formulations (14A, 14B and 14C) similar to Formula C4C but with the use of a solid emulsifier with an HLB of 14 and 4% ester oil and the addition of 4 %, 8% and 12%, respectively of fatty alcohol, were formed. All three formulations formed a stable emulsion, but each of the formulations with 8% and 12% fatty alcohol were too viscous to dispense. A sherbet foam was not formed from the formulation with 4% fatty aclcohol, although the foam exhibited a pleasant cracking sound.

### Example 15 - Personal Care Formulations with High HLB solid Emulsifier, Fatty Aclohol, Ester Oil and Silicone

A personal care formulations similar to Formula C4C but with the use of a solid emulsifier with an HLB of 15.5 (Steareth 21), 4% ester oil, 8% fatty alcohol, no water based thickener and 2% silicone were formed. The formulation formed a stable emulsion and produced a sherbet foam.

### Example16 - Personal Care Formulations with High HLB solid Emulsifier, Fatty Aclohol, Ester Oil and Silicone

A personal care formulations similar to Formula C4C but with the use of a solid emulsifier with an HLB of 15.5 (Steareth 21), 2% ester oil, 12% fatty alcohol, no water based thickener and 2% silicone were formed. The formulation formed a stable emulsion and produced a sherbet foam that exhibited a pleasant cracking sound.

### Example 17 - Personal Care Formulations with High HLB solid Emulsifier, Fatty Aclohol, Ester Oil and Silicone

A personal care formulations similar to Formula C4C but with the use of a solid emulsifier with an HLB of 15.5 (Steareth 21), 2% ester oil, 4% fatty alcohol, no water based thickener, 2% silicone and 4% of carnauba wax were formed. The formulation formed a stable emulsion and produced a sherbet foam that exhibited a pleasant cracking sound.

## Claims

1. A non-therapeutic method of treating human skin, hair and/or mucous membranes comprising:
(a) providing a personal care composition in a container under pressure in the form of a stable oil-in-water emulsion comprising:
(i) a continuous aqueous phase comprising water;
(ii) a discontinuous phase comprising foam forming component(s), trans-1-chloro-3,3,3-trifluoropropene (1233zd(E)) and trans-1-3,3,3-tetrafluoropropene (1234ze(E)), wherein said 1233zd(E) and said 1234ze (E) together comprise from about 40% to about 60% by weight of said personal care composition and are present in a trans1233zd:trans1234ze weight ratio of from about 1:8 to about 8:1;
(iii) an emulsifier having an HLB value of from about 7 to about 18 and which may be present in the continuous and/or discontinuous phase;
(iv) one or more active ingredients which may be present in the continuous and/or discontinuous phase; and
(v) optionally one or more auxiliary gel, lotion or cream forming components which may be present in the continuous and/or discontinuous phase;
(b) dispensing said personal care composition on and/or into the skin, hair and/or mucous membrane of the human being treated by discharging at least a portion of the personal care composition from said container into ambient pressure conditions to form a foam.

2. The method of claim 1 wherein said 1233zd(E) and said 1234ze (E) together comprise from about 40% to about 60% by weight of said personal care composition and are present in a trans1233zd:trans1234ze weight ratio of from about 1:8 to less than 1:1.

3. The method of claim 1 wherein said 1233zd(E) and said 1234ze (E) together comprise from about 40% to about 60% by weight of said personal care composition and are present in a trans1233zd:trans1234ze weight ratio of from about 8:1 to 1:1.

4. The method of any of claims 1 - 3 wherein said emulsifier comprises a solid emulsifier.

5. The method of any of claims 1 - 3 wherein said emulsifier is a linear, long chain solid emulsifier.

6. The method of any of claims 1 - 5 further comprising from about 9% to less than about 14% by weight of a stability assistance component.

7. The method of claim 6 wherein said stability assistance component is selected from the group consisting of ester oil, silicone, fatty alcohol, wax and combinations of two or more of these.

8. The method of claim 1 wherein said 1233zd(E) and said 1234ze (E) together comprise at least about 60% by weight of said personal care composition and are present in a trans1233zd:trans1234ze weight ratio of from about 1:8 to less than about 1:1.

9. The method of claim 1 wherein said step of forming a foam on the user provide the user with a cooling sensation and/or a crackling sound.

10. The method of any of claims 1 - 9 wherein a foam is formed on the user in a form selected from sherbet foam, ice cream foam; crackling foam or a combination of two or more of these.

## Patentansprüche

1. Nichttherapeutisches Verfahren zum Behandeln von menschlicher Haut, menschlichem Haar und/oder menschlichen Schleimhäuten, umfassend:
(a) Bereitstellen einer Körperpflegezusammensetzung in einem unter Druck stehenden Behälter in der Form einer stabilen Öl-in-Wasser-Emulsion, umfassend:
(i) eine kontinuierliche wässrige Phase, die Wasser umfasst;
(ii) eine diskontinuierliche Phase, die schaumbildende(n) Komponente(n), Trans-1-Chlor-3,3,3-Trifluorpropen (1233zd(E)) und Trans-1-3,3,3-Tetrafluorpropen (1234ze(E)) umfasst/umfassen, wobei das 1233zd(E) und das 1234ze (E) zusammen etwa 40 Gew.-% bis etwa 60 Gew.-% der Körperpflegezusammensetzung umfassen und in einem Gewichtsverhältnis Trans1233zd:Trans1234ze von etwa 1:8 bis etwa 8:1 vorliegen;
(iii) einen Emulgator mit einem HLB-Wert von etwa 7 bis etwa 18, der in der kontinuierlichen und/oder diskontinuierlichen Phase vorliegen kann;
(iv) einen oder mehrere Wirkstoffe, die in der kontinuierlichen und/oder diskontinuierlichen Phase vorliegen können; und
(v) optional ein oder mehrere Hilfsgels, Lotionen oder Cremes, die Komponenten ausbilden, die in der kontinuierlichen und/oder diskontinuierlichen Phase vorliegen können;
(b) Dispensieren der Körperpflegezusammensetzung auf und/oder in Haut, Haar und/oder Schleimhäuten des zu behandelnden Menschen durch Ausstoßen von mindestens einem Teil der Körperpflegezusammensetzung aus dem Behälter in Umgebungsdruckbedingungen, um einen Schaum auszubilden.

2. Verfahren nach Anspruch 1, wobei das 1233zd(E) und das 1234ze(E) zusammen etwa 40 Gew.-% bis etwa 60 Gew.-% der Körperpflegezusammensetzung umfassen und in einem Gewichtsverhältnis trans1233zd:trans1234ze von etwa 1:8 bis weniger als 1:1 vorliegen.

3. Verfahren nach Anspruch 1, wobei das 1233zd(E) und das 1234ze(E) zusammen etwa 40 Gew.-% bis etwa 60 Gew.-% der Körperpflegezusammensetzung umfassen und in einem Gewichtsverhältnis trans1233zd:trans1234ze von etwa 8:1 bis weniger als 1:1 vorliegen.

4. Verfahren nach Anspruch 1-3, wobei der Emulgator ein Festemulgator ist.

5. Verfahren nach Anspruch 1-3, wobei der Emulgator ein linearer, langkettiger Festemulgator ist.

6. Verfahren nach einem der Ansprüche 1-5, ferner umfassend etwa 9 Gew.-% bis weniger als etwa 14 Gew.-% einer stabilitätsunterstützenden Komponente.

7. Verfahren nach Anspruch 6, wobei die stabilitätsunterstützende Komponente ausgewählt ist aus der Gruppe bestehend aus Esteröl, Silikon, Fettalkohol, Wachs und Kombinationen aus zwei oder mehreren derselben.

8. Verfahren nach Anspruch 1, wobei das 1233zd(E) und das 1234ze(E) zusammen mindestens etwa 60 Gew.-% der Körperpflegezusammensetzung umfassen und in einem Gewichtsverhältnis trans1233zd:trans1234ze von etwa 1:8 bis weniger als etwa 1:1 vorliegen.

9. Verfahren nach Anspruch 1, wobei der Schritt des Ausbildens eines Schaums auf dem Benutzer dem Benutzer ein kühlendes Gefühl und/oder ein Knistergeräusch bereitstellt.

10. Verfahren nach einem der Ansprüche 1-9, wobei ein Schaum auf dem Benutzer in einer Form ausgebildet wird, die ausgewählt ist aus Brauseschaum, Eisschaum, Knisterschaum oder einer Kombination aus zwei oder mehreren derselben.

## Revendications

1. Procédé non thérapeutique de traitement de la peau, des cheveux et/ou des muqueuses chez l'homme comprenant :
(a) la fourniture d'une composition de soins personnels dans un récipient sous pression sous la forme d'une émulsion huile-dans-eau stable comprenant :
(i) une phase aqueuse continue comprenant de l'eau ;
(ii) une phase discontinue comprenant un (des) composant(s) formant de la mousse, du trans-1-chloro-3,3,3-trifluoropropène (1233zd(E)) et du trans-1-3,3,3-tétrafluoropropène (1234ze(E)), dans lequel ledit 1233zd(E) et ledit 1234ze(E) constituent ensemble d'environ 40 % à environ 60 % en poids de ladite composition de soins personnels et sont présents dans un rapport pondéral trans1233zd:trans1234ze d'environ 1:8 à environ 8:1 ;
(iii) un émulsifiant ayant une valeur HLB d'environ 7 à environ 18 et qui peut être présent dans la phase continue et/ou discontinue ;
(iv) un ou plusieurs principes actifs qui peuvent être présents dans la phase continue et/ou discontinue ; et
(v) éventuellement un ou plusieurs composants auxiliaires de formation de gel, de lotion ou de crème qui peuvent être présents dans la phase continue et/ou discontinue ;
(b) la distribution de ladite composition de soins personnels sur et/ou dans la peau, les cheveux et/ou la muqueuse de l'être humain traité en libérant au moins une partie de la composition de soins personnels dudit récipient dans des conditions de pression ambiante pour former une mousse.

2. Procédé selon la revendication 1, dans lequel ledit 1233zd(E) et ledit 1234ze(E) constituent ensemble d'environ 40 % à environ 60 % en poids de ladite composition de soins personnels et sont présents dans un rapport pondéral trans1233zd:trans1234ze d'environ 1:8 à moins de 1:1.

3. Procédé selon la revendication 1, dans lequel ledit 1233zd(E) et ledit 1234ze(E) constituent ensemble d'environ 40 % à environ 60 % en poids de ladite composition de soins personnels et sont présents dans un rapport pondéral trans1233zd:trans1234ze d'environ 8:1 à 1:1.

4. Procédé selon l'une quelconque des revendications 1 à 3, dans lequel ledit émulsifiant comprend un émulsifiant solide.

5. Procédé selon l'une quelconque des revendications 1 à 3, dans lequel ledit émulsifiant est un émulsifiant solide linéaire à longue chaîne.

6. Procédé selon l'une quelconque des revendications 1 à 5, comprenant en outre d'environ 9 % à moins d'environ 14 % en poids d'un composant d'aide à la stabilité.

7. Procédé selon la revendication 6, dans lequel ledit composant d'aide à la stabilité est choisi dans le groupe consistant en huile ester, silicone, alcool gras, cire et combinaisons de deux ou plus d'entre eux.

8. Procédé selon la revendication 1, dans lequel ledit 1233zd(E) et ledit 1234ze(E) constituent ensemble au moins environ 60 % en poids de ladite composition de soins personnels et sont présents dans un rapport pondéral trans1233zd:trans1234ze d'environ 1:8 à moins d'environ 1:1.

9. Procédé selon la revendication 1, dans lequel ladite étape de formation d'une mousse sur l'utilisateur procure à l'utilisateur une sensation de rafraîchissement et/ou un son de crépitement.

10. Procédé selon l'une quelconque des revendications 1 à 9, dans lequel une mousse est formée sur l'utilisateur sous une forme choisie parmi mousse de type sorbet, mousse de type crème glacée ; mousse crépitante ou une combinaison de deux ou plus d'entre elles.
